# EUROPEAN PATENT APPLICATION

(11) **EP 4 000 622 A1**
(43) Date of publication of application: **25.05.2022**
(21) Application number: 20841440.9
(22) Date of filing: 14.07.2020
(51) Int. Cl.: A61K 31/495, A61P 19/00, A23L 33/10

(54) **PHARMACEUTICAL COMPOSITION FOR PREVENTING OR TREATING BONE DISEASES**

(30) Priority: 15.07.2019 KR 20190084955; 13.07.2020 KR 20200086332
(71) Applicant: Industry Foundation of Chonnam National University, Gwangju 61186 (KR)
(72) Inventor: YOON, Sung Wook, Suwon-si, Gyeonggi-do 16534 (KR); LEE, Seung Won, Gwangmyeong-si, Gyeonggi-do 14293 (KR); HAN, Dae Nam, Gyeonggi-do 34020 (KR); KIM, Yong Hwan, Anyang-si, Gyeonggi-do 13970 (KR)
(74) Representative: Schön, Christoph
(86) International application number: PCT/KR2020/009246
(87) International publication number: WO 2021/010728

(57) **Abstract**

The present invention relates to a pharmaceutical composition for preventing or treating bone diseases, and more specifically, the pharmaceutical composition includes a compound represented by a specific chemical formula so as to inhibit osteoclast differentiation and/or formation and exhibit excellent prophylactic or therapeutic effects on various bone diseases including periodontitis and the like.

<110>     INDUSTRY FOUNDATION OF CHONNAM NATIONAL UNIVERSITY

<120>     Pharmaceutical compositions for preventing or treating bone
          diseases

<130>     20OP06013PCT

<150>     KR 10-2019-0084955
<151>     2019-07-15

<150>     KR 10-2020-0086332
<151>     2020-07-13

<160>     12

<170>     KoPatentIn 3.0

<210>     1
<211>     20
<212>     DNA
<213>     Artificial Sequence

<220>
<223>     NFATc1 Forward primer


<400>     1
cccgtcacat tctggtccat                                                   20


<210>     2
<211>     23
<212>     DNA
<213>     Artificial Sequence

<220>
<223>     NFATc1 Reverse primer


<400>     2
caagtaaccg tgtagctcca caa                                               23


<210>     3
<211>     21
<212>     DNA
<213>     Artificial Sequence

<220>
<223>     CatK Forward primer


<400>     3
ggacgcagcg atgctaacta a                                                 21


<210>     4
<211>     28
<212>     DNA
<213>     Artificial Sequence

<220>
<223>     CatK Reverse primer

<400>	4
cagagagaag ggaagtagag ttgtcact	28

<210>	5
<211>	21
<212>	DNA
<213>	Artificial Sequence

<220>
<223>	c-Fos Forward primer

<400>	5
cgaagggaac ggaataagat g	21

<210>	6
<211>	20
<212>	DNA
<213>	Artificial Sequence

<220>
<223>	c- Fos Reverse primer

<400>	6
gctgccaaaa taaactccag	20

<210>	7
<211>	20
<212>	DNA
<213>	Artificial Sequence

<220>
<223>	DC-STAMP Forward primer

<400>	7
gggagtcctg caccatatgg	20

<210>	8
<211>	22
<212>	DNA
<213>	Artificial Sequence

<220>
<223>	DC-STAMP Reverse primer

<400>	8
aggccagtgc tgactaggat ga	22

<210>	9
<211>	23
<212>	DNA
<213>	Artificial Sequence

<220>
<223>    OC-STAMP Forward primer

<400>    9
cagagtgacc acctgaacaa aca                                               23

<210>    10
<211>    20
<212>    DNA
<213>    Artificial Sequence

<220>
<223>    OC-STAMP Reverse primer

<400>    10
tgcctgaggt ccctgtgact                                                   20

<210>    11
<211>    22
<212>    DNA
<213>    Artificial Sequence

<220>
<223>    TRAF6 Forward primer

<400>    11
aaagcgagag attctttccc tg                                                22

<210>    12
<211>    22
<212>    DNA
<213>    Artificial Sequence

<220>
<223>    TRAF6 Reverse primer

<400>    12
actggggaca attcactaga gc                                                22

## Description

### [Technical Field]

The present invention relates to a pharmaceutical composition and a health functional food for preventing or treating bone diseases.

### [Background Art]

Bone tissue is a tissue in which bone resorption by osteoclasts and bone formation by osteoblasts are continuously maintained. Differentiation of osteoblasts is promoted by signal factors such as bone morphogenetic protein 2 (BMP2). On the other hand, differentiation of osteoclasts is promoted by receptor activator of nuclear factor kappa-β ligand (RANKL) signaling substances produced in the osteoblast differentiation stage, and apoptosis occurs when the differentiation is completed. Therefore, it is important to harmonize the differentiation and activity of the osteoblasts and osteoclasts during normal bone regeneration.

Periodontal inflammation is an inflammatory response by the defense of immune cells against apical bacterial infection. Neutrophils, which are mainly involved in periodontal inflammation, secrete prostaglandins as an inflammatory mediator. Cellular signaling substances such as prostaglandin and RANKL can activate osteoclasts that absorb bone tissue and cause resorption of alveolar bone around the inflamed area. For the treatment of chronic periodontitis, it is required to develop bone regeneration promoters or bone resorption inhibitors. In addition, in order to control abnormal bone resorption and restore normal bone regeneration processes, it is required to develop osteoclast differentiation inhibitors.

### [Summary of Invention]

### [Problems to be Solved by Invention]

An object of the present invention is to provide a pharmaceutical composition for preventing or treating bone diseases.

Another object of the present invention is to provide a health functional food for preventing or improving bone diseases.

### [Means for Solving Problems]

1. A pharmaceutical composition for preventing or treating bone diseases caused by hyperdifferentiation of osteoclasts, the pharmaceutical composition including a compound represented by Formula 1 below or a pharmaceutically acceptable salt thereof: 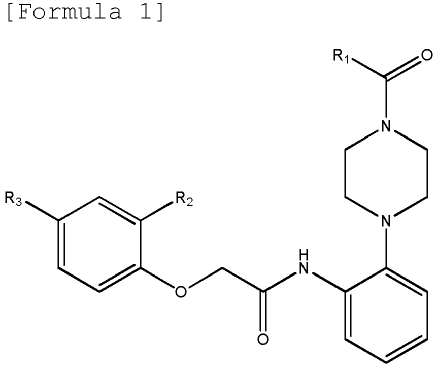 In Formula 1 above,
   R₁ is C1 to C3 alkyl or phenyl; and
   R₂ and R₃ are each independently H or halo.
2. The pharmaceutical composition according to the above 1, wherein R₁ is methyl, ethyl or phenyl.
3. The pharmaceutical composition according to the above 1, wherein R₁ is C1 to C3 alkyl, R₂ is halo and R₃ is H; or R₁ is phenyl, R₂ is H and R₃ is halo.
4. The pharmaceutical composition according to the above 1, wherein the compound represented by Formula 1 above is any compound represented by Formulae 2 to 4 below: 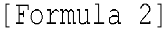 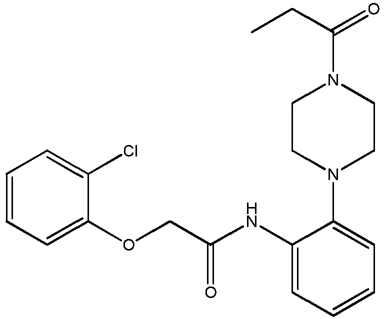 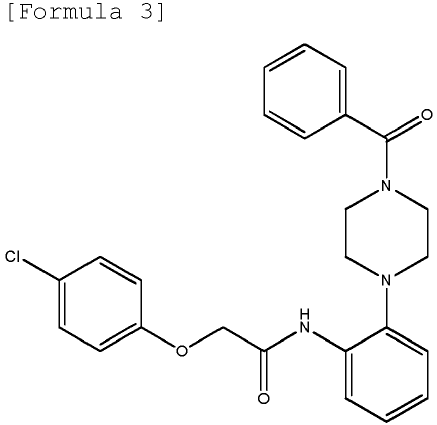 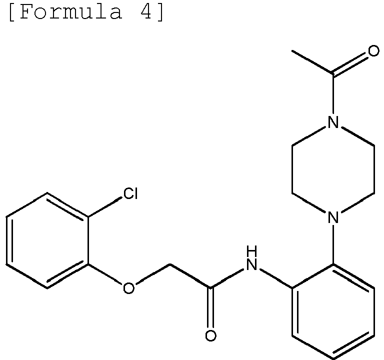
5. The pharmaceutical composition according to the above 1, wherein the bone disease is at least one selected from the group consisting of fracture, osteoporosis, rheumatoid arthritis, periodontitis, Paget's disease, osteomalacia, osteopenia, bone atrophy, osteoarthritis, and avascular femoral necrosis.
6. A health functional food for preventing or treating bone diseases caused by hyperdifferentiation of osteoclasts, the health functional food including a compound represented by Formula 1 below or a food acceptable salt thereof: 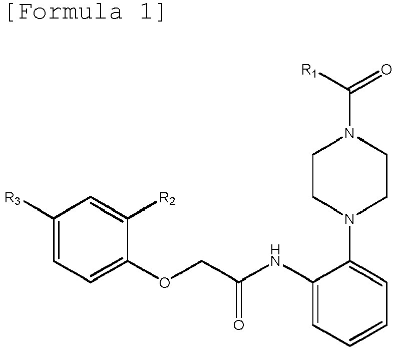 In Formula 1 above,
   R₁ is C1 to C3 alkyl or phenyl; and
   R₂ and R₃ are each independently H or halo.
7. The health functional food according to the above 6, wherein R₁ is methyl, ethyl or phenyl.
8. The health functional food according to the above 6, wherein R₁ is C1 to C3 alkyl, R₂ is halo and R₃ is H; or R₁ is phenyl, R₂ is H and R₃ is halo.
9. The health functional food according to the above 6, wherein the compound represented by Formula 1 above is any compound represented by Formulae 2 to 4 below: 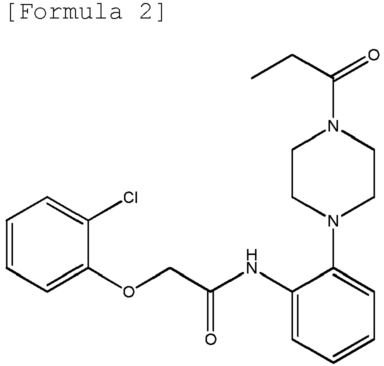 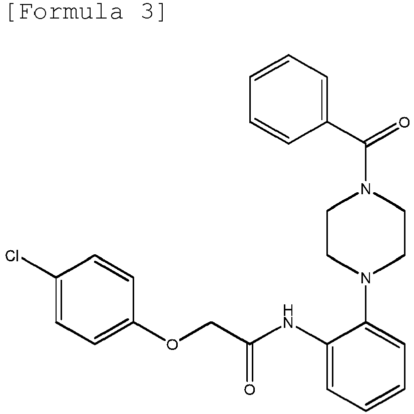 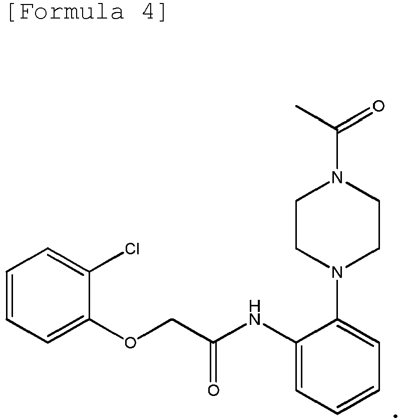
10. The health functional food according to the above 6, wherein the bone disease is at least one selected from the group consisting of fracture, osteoporosis, rheumatoid arthritis, periodontitis, Paget's disease, osteomalacia, osteopenia, bone atrophy, osteoarthritis, and avascular femoral necrosis.

### [Advantageous Effects]

The pharmaceutical composition of the present invention may inhibit differentiation and/or production of osteoclasts, thereby exhibiting excellent prophylactic or therapeutic effects for various bone diseases including periodontitis.

The health functional food of the present invention may inhibit differentiation and/or production of osteoclasts, thereby exhibiting excellent preventive or improvement effects for various bone diseases including periodontitis.

### [Brief Description of Drawings]

FIG. 1 shows results of confirming the effect of a compound on osteoclast differentiation ability through tartrate-resistant acid phosphatase (TRAP) staining.
FIG. 2 is a graph showing an osteoclast differentiation area ratio and the number of differentiated osteoclasts per total area confirmed after TRAP staining.
FIG. 3 shows results of a cytotoxicity test of 35D35 compound through an MTT analysis.
FIG. 4 shows results of confirming whether the 35D35 compound is effective depending on osteoclast differentiation timing through TRAP staining.
FIG. 5 shows results of confirming F-actin formation inhibitory ability of 35D35, 35D5, and 35D3 compounds.
FIG. 6 shows results of measuring F-actin formation inhibitory ability of the 35D35 compound by treatment concentration.
FIG. 7 shows results of measuring a bone resorption capacity of 35D35 using a bone resorption assay kit.
FIG. 8 shows results of confirming an expression level of osteoclast-specific gene through RT-PCR.
FIG. 9 shows results of comparing an amount of CtsK expression in osteoclasts treated with the 35D35 compound (1 µM) and 35D3 compound (6 µM) through RT-PCR, respectively.
FIG. 10 shows ALP staining results of confirming whether the 35D35 compound is effective on the osteoblast differentiation.
FIG. 11 shows results of confirming effects of increasing bone mineral density by the 35D35, 35D5, and 35D3 compounds, respectively, in OVX mice.
FIG. 12 shows trabecular bone mineral density (BMD) values in OVX mice treated with the 35D35, 35D5 and 35D3 compounds, respectively.
FIG. 13 shows results of confirming effects of increasing the bone mineral density by the 35D35 compound in OVX mice.
FIG. 14 shows values of a total bone volume (TV), bone volume (BV), trabecular volume (Th. V), trabecular bone mineral density (Th. BMD), cortical volume (Ct. V), cortical bone mineral density (Ct. BMD) in OVX mice treated with the 35D35 compound.
FIG. 15 shows results of assessing cell viability when mononuclear cells isolated from mouse bone marrow were treated with the 35D3 compounds at different concentrations.
FIG. 16 shows results of confirming effects of the 35D3 compound on the osteoclast differentiation ability using a tartrate-resistant acid phosphatase (TRAP) activity assay kit (Sigma-Aldrich, 387A-1kt).
FIG. 17 shows a differentiation area ratio per total area and the number of differentiated osteoclasts confirmed after TRAP staining.
FIG. 18 shows results of confirming the degree of formation of F-actin for differentiation into multinuclear cells.
FIG. 19 shows results of confirming the bone resorption ability of osteoclasts.

### [Mode for Carrying out Invention]

Hereinafter, the present invention will be described in more detail.

The present invention provides a pharmaceutical composition for preventing or treating bone diseases, which includes a compound represented by Formula 1 below or a pharmaceutically acceptable salt thereof. 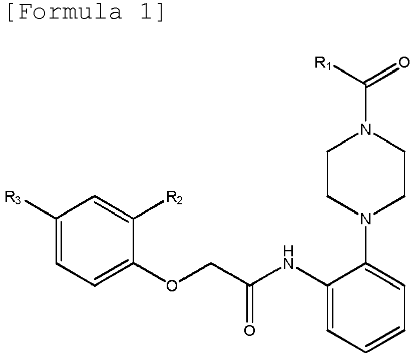 In Formula 1 above,
R₁ is C1 to C3 alkyl or phenyl; and
R₂ and R₃ are each independently H or halo.

The term "alkyl" refers to a linear or branched saturated hydrocarbon group, such as methyl, ethyl, propyl, isopropyl, isobutyl, sec-butyl, tert-butyl, pentyl, hexyl, heptyl, octyl, nonyl, decyl, undecyl, tridecyl, pentadecyl and heptadecyl and the like. C1 to C10 alkyl means alkyl having 1 to 10 carbon atoms.

The term "aryl" refers to a wholly or partially unsaturated and substituted or unsubstituted monocyclic or polycyclic carbon ring, and may be, for example, substituted or unsubstituted phenyl.

The term "halo" refers to a monovalent functional group of elements belonging to group 17 in the periodic table, and may be, for example, fluoro, chloro, bromo or iodo.

In Formula 1, R₁ may be C1 to C3 alkyl or phenyl.

In Formula 1, R₁ may be methyl, ethyl or phenyl.

In Formula 1, R₂ and R₃ may be each independently H or halo.

In Formula 1, R₂ and R₃ may be each independently H or chloro.

In Formula 1, R₁ may be C1 to C3 alkyl, R₂ may be halo, and R₃ may be H.

According to an embodiment, in Formula 1, R₁ may be ethyl, R₂ may be chloro, and R₃ may be H.

According to an embodiment, in Formula 1, R₁ may be methyl, R₂ may be chloro, and R₃ may be H.

In Formula 1, R₁ may be phenyl, R₂ may be H, and R₃ may be halo.

According to an embodiment, in Formula 1, R₁ may be phenyl, R₂ may be H, and R₃ may be chloro.

The compound represented by Formula 1 may be selected from the group consisting of compounds represented by Formulae 2 to 4 below.

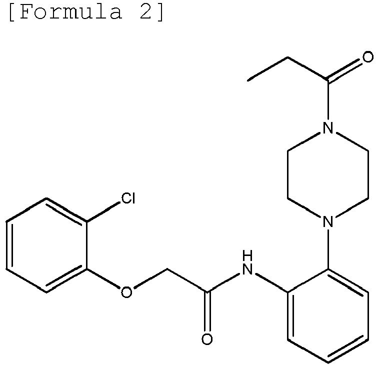

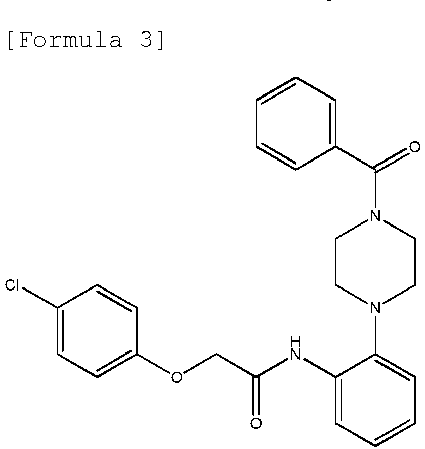

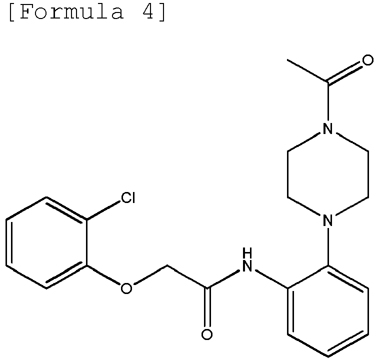

According to an embodiment, a pharmaceutical composition for preventing or treating bone diseases, which includes a compound represented by each of Formulae 2 to 4, or a pharmaceutically acceptable salt thereof may be provided.

The term "pharmaceutically acceptable" refers to a feature that does not cause serious irritation to an individual, cells, tissues, etc. to which a compound or composition is administered, and does not impair biological activity and physical properties of the compound.

Pharmaceutically acceptable salts may be, for example, acid addition salts, base addition salts or metal salts.

The acid addition salts may be formed from inorganic acids such as hydrochloric acid, nitric acid, phosphoric acid, sulfuric acid, hydrobromic acid, hydroiodic acid, nitrous or phosphorous acid, aliphatic mono and dicarboxylates, phenyl-substituted alkanoates, hydroxy alkanoates and alkane dioates, and non-toxic organic acids such as aromatic acids, aliphatic and aromatic sulfonic acids. These pharmaceutically non-toxic salts may include sulfate, pyrosulfate, bisulfate, sulfite, bisulfite, nitrate, phosphate, monohydrogen phosphate, dihydrogen phosphate, metaphosphate, pyrophosphate, chloride, bromide, iodide, fluoride, acetate, propionate, decanoate, caprylate, acrylate, formate, isobutyrate, caprate, heptanoate, propyolate, oxalate, malonate, succinate, suberate, sebacate, fumarate, maleate, butine-1,4-dioate, nucleic acid-1,6-dioate, benzoate, chlorobenzoate, methylbenzoate, dinitrobenzoate, hydroxybenzoate, methoxybenzoate, phthalate, terephthalate, benzene sulfonate, toluene sulfonate, chlorobenzene sulfonate, xylene sulfonate, phenylacetate, phenylpropionate, phenylbutyrate, citrate, lactate, β_hydroxybutyrate, glycolate, malate, tartrate, methane sulfonate, propane sulfonate, naphthalene-1-sulfonate, naphthalene-2-sulfonate or mandelate. For example, the acid addition salt of the compound represented by Formula 1 may be obtained by dissolving the compound in an excess amount of aqueous acid solution and precipitating the salt using a hydrated organic solvent such as methanol, ethanol, acetone or acetonitrile.

The metal salt may be a sodium, potassium or calcium salt. The metal salt may be prepared using a base, for example, alkali-metal or alkaline earth metal salts may be obtained by dissolving the compound in an excess amount of alkali-metal hydroxide or alkaline earth metal hydroxide solution, filtering the non-dissolved compound salt, and evaporating and/or drying the filtrate.

The compound represented by Formula 1 and a pharmaceutically acceptable salt thereof may exhibit osteoclast differentiation inhibitory effects.

The compound represented by each of Formulae 2 to 4, and a pharmaceutically acceptable salt thereof, may exhibit osteoclast differentiation inhibitory effects.

The term "prevention" refers to any action that inhibits or delays bone disease.

The term "treatment" refers to any action that improves or beneficially alters symptoms of an individual suspected of and developed bone disease.

The compounds represented by Formulae 1 to 4 included in the composition of the present invention may be derived from nature or may be synthesized using a known chemical synthesis method.

Bone diseases that can be prevented or treated by the pharmaceutical composition of the present invention may be caused by an imbalance of activities between osteoblasts and osteoclasts.

Since bone is a living tissue, old bone is destroyed regularly and undergoes a reformation process to create new bones. In this process, osteoclasts destroy old and unnecessary bone tissues, and calcium is released into the blood stream to help maintain body functions, while osteoblasts play a role of regenerating the destroyed bone. This reaction continues 24 hours a day, and about 10% to 30% of adult bones are regenerated in this way every year. Therefore, the balance between the osteoclasts and the osteoblasts is very important, and this balance is regulated by various hormones and other body chemicals.

Specifically, the bone disease may be caused by osteoclast hyperdifferentiation or decrease in osteoblast activity, and specifically, the bone disease may be caused by osteoclast hyperdifferentiation.

When the osteoclasts are hyperdifferentiated, the osteoclasts may be increased abnormally and cause excessive bone resorption, thereby resulting in lower bone density. For example, various diseases such as osteoporosis, osteomalacia, osteopenia, bone atrophy, and periodontitis may be developed.

The bone diseases may include, for example, fracture, osteoporosis, rheumatoid arthritis, periodontitis, Paget's disease, osteomalacia, osteopenia, bone atrophy, osteoarthritis or avascular femoral necrosis, bone defects, fracture osteoporotic fractures, diabetic fractures, nonunion fractures, bone insufficiency, osteoporotic fracture, bone dysplasia, degenerative bone disease, malunion, bone union disorder, arthrosis, bone necrosis, osteoarthritis, bone tumor, bone cancer, etc., but it is not limited thereto. Preferably, the bone disease may be fracture, osteoporosis, rheumatoid arthritis, periodontitis, Paget's disease, osteomalacia, osteopenia, bone atrophy, osteoarthritis or avascular femoral necrosis, but it is not limited thereto.

Periodontitis is an inflammatory reaction caused by the defense of immune cells against apical bacterial infection. Neutrophils, which are mainly involved in periodontitis, secrete prostaglandins as an inflammatory mediator. The osteoclasts absorbing bone tissues are activated by cellular signaling substances such as prostaglandin, and the alveolar bone loss is observed around the inflamed area. Further, chronic periodontitis shows persistent inflammation in the apical region of the periodontal bone and erosion of the alveolar bone. If periodontitis becomes worsen and teeth cannot be saved, tooth extraction and implantation are performed. In order to prevent such conditions as described above, antibiotics should be administered to reduce the number of cells causing infection and osteoclast inhibitors are further administered to overcome the activation of osteoclasts by inflammatory mediators in the early stages of periodontitis, whereby alleviation and treatment of chronic periodontitis may be expected.

The pharmaceutical composition for preventing or treating bone diseases of the present invention may be used for the prevention or treatment of periodontitis based on bone resorption inhibitory effects.

The pharmaceutical composition of the present invention may be admixed and provided with known bone disease treatment substances.

The pharmaceutical composition of the present invention may be administered in combination with known substances for preventing or treating bone diseases.

The term "administration" refers to introducing a predetermined substance to an individual by an appropriate method, and the term "subject" refers to all animals such as rats, mice, livestock, as well as humans who have or may develop bone disease. As a specific example, it may be a mammal including a human.

If necessary, the pharmaceutical composition of the present invention may additionally include a known anti-bone disease compound.

Examples of these anti-bone disease compounds may include, for example, cinchonin, extracts of brown mealworm, aloe-emodin and omega-3 fatty acids, arteannnuin B, indole-2-carboxylate derivatives, euphrobia factor L1, scalcapflavon derivatives, and praxinelone, etc., but it is not limited thereto.

The pharmaceutical composition of the present invention may be in the form of a capsule, tablet, granule, injection, ointment, powder or beverage.

The pharmaceutical composition of the present invention may be formulated and used in oral dosage forms such as powder remedies, granules, capsules, tablets, and aqueous suspensions, external preparations, suppositories and injections.

The pharmaceutical composition of the present invention may contain an active ingredient alone, or may further include one or more pharmaceutically acceptable carriers, excipients, or diluents.

The pharmaceutical composition of the present invention may include a pharmaceutically acceptable carrier. The pharmaceutically acceptable carrier may be binders, lubricants, disintegrants, excipients, solubilizers, dispersants, stabilizers, suspending agents, coloring agents, flavoring agents, etc. for oral administration. Further, for injections, a buffering agent, a preservative, a soothing agent, a solubilizing agent, an isotonic agent, a stabilizer, etc. may be mixed and used. Further, for topical administration, a base agent, an excipient, a lubricant, a preservative, etc. may be used.

The formulation of the pharmaceutical composition of the present invention may be prepared in various ways by mixing the composition with the pharmaceutically acceptable carrier. For example, when administered orally, it may be prepared in the forms of tablets, troches, capsules, elixir, suspension, syrup, wafers, etc. Further, in the case of an injection, it may be prepared in a unit dosage ampoule or a multiple dosage form. In addition, the formulation of the pharmaceutical composition of the present invention may be prepared as a solution, suspension, tablet, capsule, sustained release formulation, or the like.

The carriers, excipients and diluents for formulation may include, for example, lactose, dextrose, sucrose, sorbitol, mannitol, xylitol, erythritol, malditol, starch, gum acacia, alginate, gelatin, calcium phosphate, calcium silicate, cellulose, methyl cellulose, microcrystalline cellulose, polyvinylpyrrolidone, water, methylhydroxybenzoate, propylhydroxybenzoate, talc, magnesium stearate, mineral oil, filler, anti-coagulant, lubricant, wetting agent, fragrance, emulsifier or preservative.

The administration route of the pharmaceutical composition of the present invention may include, for example oral, intravenous, intramuscular, intraarterial, intramedullary, intrathecal, intracardiac, transdermal, subcutaneous, intraperitoneal, intranasal, intestinal, topical, sublingual or rectal, but it is not limited thereto.

The pharmaceutical composition of the present invention may be administered orally or parenterally and, when administered parenterally, external preparations or injection methods such as intraperitoneal, rectal, subcutaneous, intravenous, intramuscular or intrathoracic injection may be selected.

The dosage of the pharmaceutical composition of the present invention may vary depending on the condition and weight of the patient, the degree of the disease, the form of the drug, the route and duration of administration, but may be appropriately selected by those skilled in the art.

For example, the pharmaceutical composition of the present invention may be administered at 0.0001 to 1000 mg/kg or 0.001 to 500 mg/kg per day. Further, the pharmaceutical composition of the present invention may be administered once a day, or may be divided several times.

In addition, the present invention provides a health functional food for preventing or improving bone diseases, which includes a compound represented by Formula 1, or a food acceptable salt thereof: 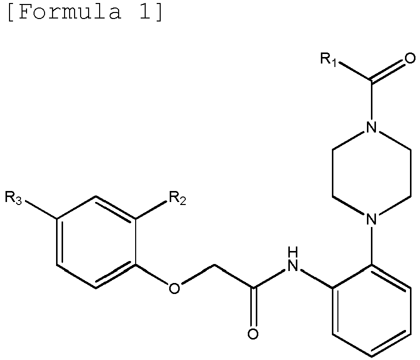 In Formula 1 above,
R₁ is C1 to C3 alkyl or phenyl; and
R₂ and R₃ are each independently H or halo.

According to an embodiment, it is possible to provide a health functional food for preventing or improving bone disease, which includes a compound represented by each of Formulae 2 to 4, or a food acceptable salt thereof.

Since the compounds represented by Formulae 1 to 4 and the bone diseases have been described above, therefore will not be described in detail.

The health functional food of the present invention may be formulated as one selected from the group consisting of tablets, pills, powder remedies, granules, powders, capsules, and liquid formulations by further adding at least one of carriers, diluents, excipients and additives thereto.

The health functional food may be, for example, various foods, powders, granules, tablets, capsules, syrup, beverages, gums, teas, vitamin complexes, or health functional foods.

Additives that can be included in health functional foods may be selected from the group consisting of natural carbohydrates, flavoring agents, nutrients, vitamins, minerals (electrolytes), flavoring agents (synthetic flavors, natural flavors, etc.), coloring agents, fillers (cheese, chocolate, etc.), pectic acid or salts thereof, alginic acid and salts thereof, organic acids, protective colloidal thickeners, pH adjusters, stabilizers, preservatives, antioxidants, glycerin, alcohols, carbonation agents and pulp.

Examples of natural carbohydrates may be: monosaccharides such as glucose, fructose, and the like; disaccharides such as maltose, sucrose, and the like; and polysaccharides, for example, common sugars such as dextrin and cyclodextrin, and sugar alcohols such as xylitol, sorbitol, and erythritol and the like. Further, as the flavoring agent, natural flavors (taumatin, stevia extract (e.g., rebaudioside A, glycyrrhizin, etc.)), and synthetic flavors (saccharin, aspartame, etc.) may be advantageously used.

The health functional food may further include various nutrients, vitamins, minerals (electrolytes), flavoring agents such as synthetic flavors and natural flavors, coloring agents and thickeners (cheese, chocolate, etc.), pectic acid and salts thereof, alginic acid and salts thereof, and organic acids, protective colloidal thickeners, pH adjusters, stabilizers, preservatives, glycerin, alcohol, carbonation agents used in carbonated beverages, and pulp for production of natural fruit juices and vegetable beverages and the like.

The carriers, excipients, diluents and additives are not limited thereto, but may be selected from the group consisting of lactose, dextrose, sucrose, sorbitol, mannitol, erythritol, starch, gum acacia, calcium phosphate, alginate, gelatin, calcium phosphate, calcium silicate, microcrystalline cellulose, polyvinylpyrrolidone, cellulose, polyvinylpyrrolidone, methylcellulose, water, sugar syrup, methylcellulose, methyl hydroxy benzoate, propylhydroxy benzoate, talc, magnesium stearate and mineral oil.

When formulating the health functional food of the present invention, it may be prepared using diluents or excipients such as fillers, extenders, binders, wetting agents, disintegrants, and surfactants.

Hereinafter, the present invention will be more specifically described by way of the following examples.

In the following examples, 35D35 is a compound represented by Formula 2 above, that is, (2-(2-chlorophenoxy)-N-[2-(4-propionyl-1-piperazinyl)phenyl]acetamide), 35D5 is a compound represented by Formula 3 above, 35D3 is N-(2-(4-acetylpiperazin-1-yl)phenyl)-2-(2-chlorophenoxy)acetamide, 35D8 is 2-(4-chlorophenoxy)-N-(2-(4-methylpiperazin-1-yl)phenyl)acetamide, and 35D3 (or PPOA) is a compound represented by Formula 4 above.

### MTT assay for cytotoxicity assessment

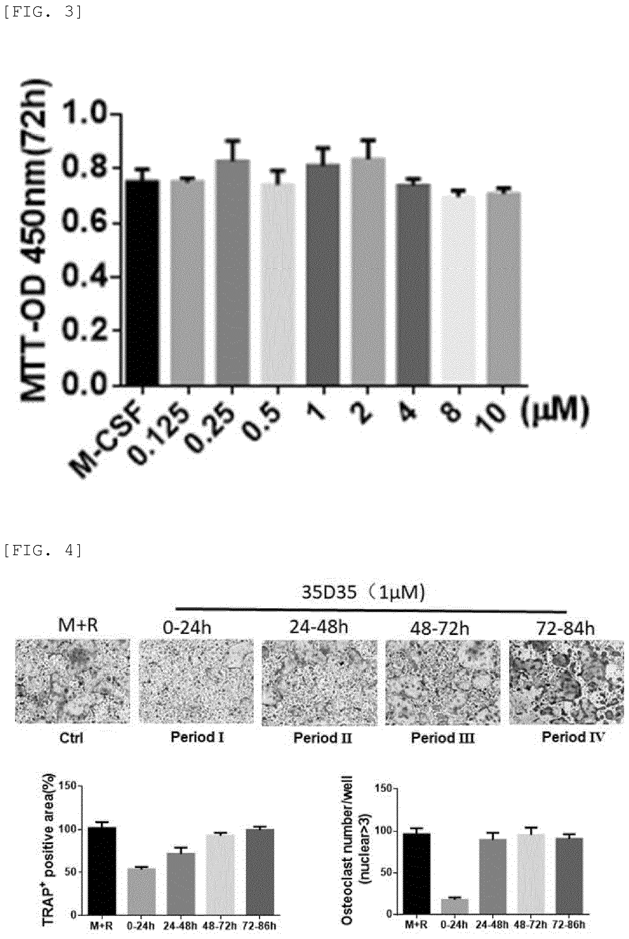

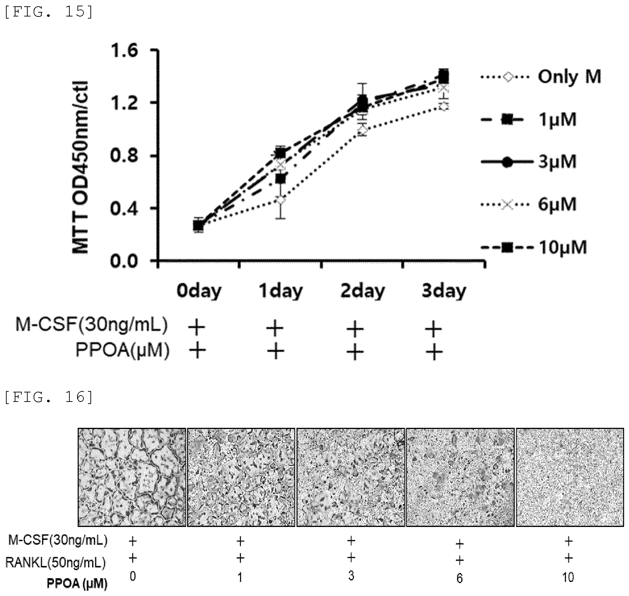

After treatment of preosteoclasts with 35D35 or 35D3 (=PPOA) (1-10 uM), cell viability was evaluated. Specifically, 1 × 10⁴ cells per well were put into a 96-well plate, and only MCSF (30 ng/mL) and PPOA were cultured by day under the indicated concentration conditions. Thereafter, a cell viability assay reagent (DoGen, EZ-3000) was used with 1/10 of the volume of the medium for treatment, and then the cells were incubated at 37°C for 30 minutes and observed at 450 nM. As a result of confirming the cytotoxicity using the 35D35 compound and PPOA, even when the concentration of 10 uM was used for treatment, apoptosis effects were not exhibited (see FIGS. 3 and 15). That is, it was confirmed that there was no cytotoxicity.

### Confirmation of osteoclast differentiation inhibitory effect

### 1. Inhibitory effects of the compound represented by Formulae 2 and 3 on osteoclast differentiation

### 1) Isolation of mouse bone marrow cells and differentiation of osteoclasts

The bone marrow of a 10-week-old female mouse was isolated from the hip bone, femur and tibia, followed by culturing the same in α-MEM medium (Gibco, 12561-056, 10% fetal bovine serum, 1% penicillin/streptomycin) containing macrophage colony stimulating factor (M-CSF, PeproTech, 315-02, 30 ng/ml) for 3 days in order to isolate only monocytes. The isolated monocytes were treated with receptor activator of nuclear factor kappa B ligand (RANKL, PeproTech, 315-11, 50 ng/ml) to induce osteoclast differentiation. During RANKL treatment, each compound (35D35, 35D5, 35D3, and 35D8) was used for treatment and the cells were cultured for 3 or 5 days so as to confirm the degree of osteoclast differentiation.

### 2) Confirmation of osteoclast differentiation inhibitory ability through TRAP staining and assay

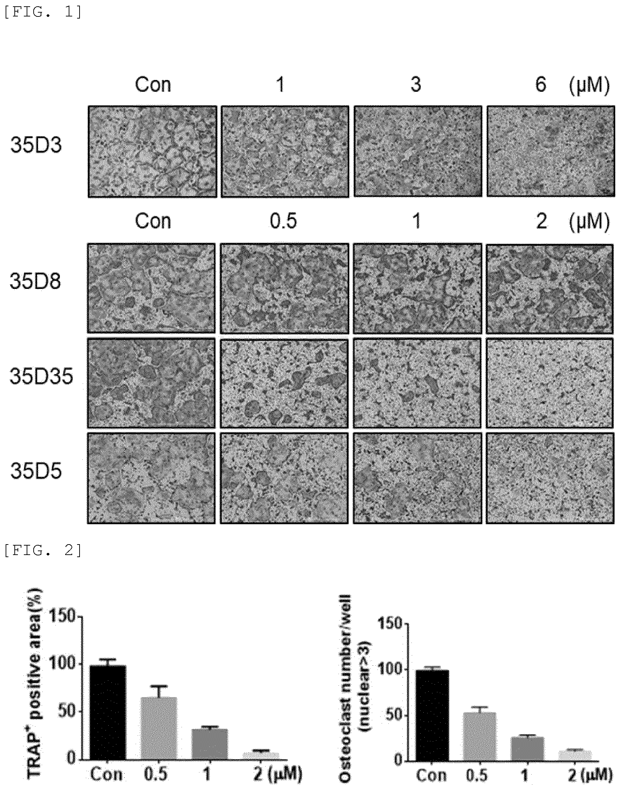

1 × 10⁴ monocytes isolated from the mouse bone marrow were put into a 96-well plate and treated with each compound (35D35, 35D5, 35D3, and 35D8) together with RANKL, thereby confirming the effects of each compound on osteoclast differentiation ability. Using the TRAP activity assay kit (Cosmo Bio, PMC-AK04F-COS), the activity of tartrate-resistant acid phosphatase (TRAP) with increased expression during osteoclast differentiation was confirmed. As a result of confirming the degree of osteoclast differentiation by TRAP staining, the compounds 35D35, 35D5 and 35D3 exhibited superior osteoclast differentiation inhibitory effects compared to other derivatives (see FIG. 1). After staining, multinuclear cells formed during osteoclast differentiation were confirmed under a microscope. Further, each well was photographed and a differentiation area ratio per total area was determined using Image J software. As a result, the 35D35 compound showed an effective osteoclast differentiation inhibitory ability at a concentration of 0.5 uM (see FIG. 2).

Further, the effects of the 35D35 compound having excellent osteoclast production inhibitory ability with respect to osteoclast differentiation period was investigated. Specifically, BMM was divided into 5 groups (Ctrl, Period I to IV), and cultured for 4 days in a culture medium containing 30 ng/mL M-CSF and 50 ng/mL RANKL. BMMs in Period I to IV groups were exposed to 1 µM 35D35 for 24 hours on different days, respectively. After 4 days, the cells of each group were fixed, followed by TRAP staining, and then, the presence of osteoclasts was confirmed. As a result, it was confirmed that osteoclast differentiation was inhibited for the initial 24 hours in the group treated with the 35D35 compound (see FIG. 4, "M+R" means M-CSF+RANKL treatment).

From the above results, it was confirmed that the 35D35, 35D5 and D35D3 compounds exhibited excellent osteoclast differentiation inhibitory effects, and particularly, were effective at the early stage of osteoclast differentiation.

### 3) Assessment of bone resorption inhibition ability

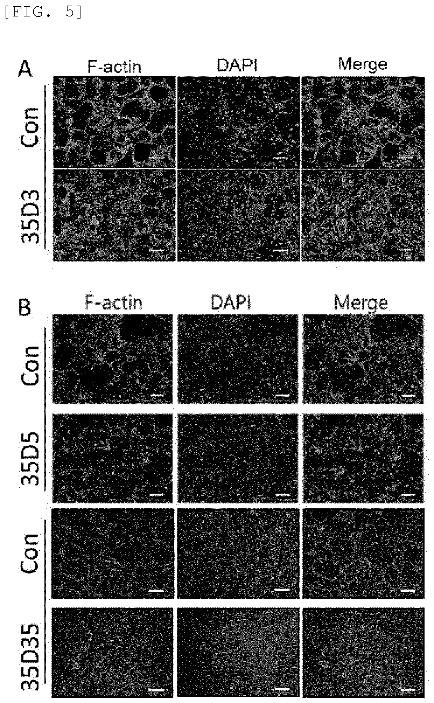

In order to confirm the activity of differentiated osteoclasts, F-actin ring formation ability was measured. Specifically, BMM was seeded on a 12 mm cover glass with or without compounds (35D3: 6 µM, 35D5 and 35D35: 2 µM), followed by M-CSF and LANKL treatment. After the osteoclasts were formed in the control, the osteoclasts were fixed in 4.0% paraformaldehyde for 15 minutes, and then incubated in 5% FBS and blocked for about 60 minutes. After washing with PBS, rhodamine-conjugated phalloidin (1:40) was added to each well to visualize a F-actin belt. After 20 minutes, the cells were incubated along with a DAPI (1:5000) solution for 5 minutes. After washing three times with PBS, cells were observed using a fluorescence microscope. As a result, 35D35, 35D5 and 35D3 showed excellent effects of inhibiting RANKL-induced F-actin belt formation (see FIG. 5, scale bar = 200 µm, "Con" = M-CSF+RANKL treatment group).

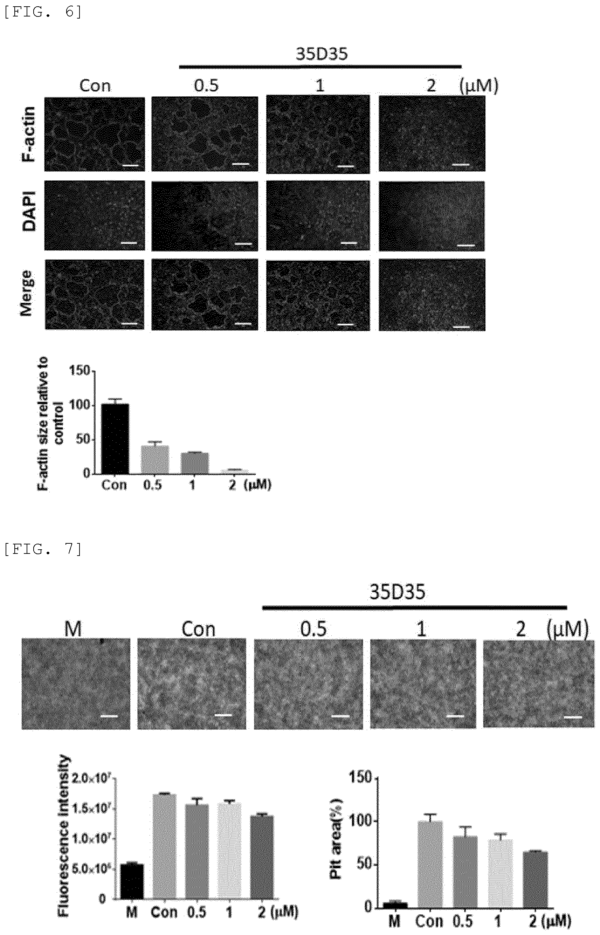

Further, as a result of confirming the F-actin ring formation ability by treatment using the 35D35 compound, which exhibits excellent F-actin belt formation inhibitory effects at different concentrations, it was confirmed that the F-actin ring formation ability was decreased by 50% or more in the cells treated with 0.5 uM of 35D35 (see FIG. 6, scale bar = 200 µm, "Con" = M-CSF+RANKL treatment group, "M" = M-CSF treatment group). A F-actin size was measured using Image J.

In addition, the bone resorption ability of 35D35 was measured using a bone resorption assay kit (Cosmo Bio, CSR-BRA). Specifically, BMM was seeded on a fluoresceinamine-labeled calcium phosphate plate, followed by 35D35 treatment with various doses. After 6 days, a fluorescence intensity was measured at absorption and emission wavelengths of 485 nm and 535 nm, respectively, using a fluorescent reader. Further, a resorption pit area was calculated using Image J. As a result, it was confirmed that the bone resorption ability was reduced by about 10% in the cells treated with 0.5 uM of 35D35, as compared to the control cells not treated with the compound (Ctrl) (see FIG. 7, scale bar = 200 µm, "Con" = M -CSF+RANKL treatment group, "M" = M-CSF treatment group).

### 4) Confirmation of decreased expression of transcription factors related to osteoclast differentiation

In order to confirm the expression degree of the osteoclast-specific gene, an mRNA expression level was measured by RT-PCR in cells treated with 35D35 (1 µM) and 35D3 (6 µM) compounds, respectively. RNA was isolated from cells differentiated into osteoclasts using an RNA extraction kit, and cDNA was synthesized from 0.5 µg of RNA, which was quantified through a spectrophotometer, using a reverse transcriptase (Takara, RR037 A). Thereafter, QRT-PCR was performed using cDNA synthesized by QuantStudio 3 real-time PCR system (Applied Biosystems). After treating the cells with lysates, immunoblotting was conducted with an anti-Ctsk antibody. At this time, β-actin was used as a loading control.

The primers used in qRT-PCR are shown in Table 1 below.

**[TABLE 1]**

| Primer name | Sequence | Sequence number |
|---|---|---|
| NFATcl Forward primer | CCCGTCACATTCTGGTCCAT | SEQ ID NO: 1 |
| NFATcl Reverse primer | CAAGTAACCGTGTAGCTCCACAA | SEQ ID NO: 2 |
| CatK Forward primer | GGACGCAGCGATGCTAACTAA | SEQ ID NO: 3 |
| CatK Reverse primer | CAGAGAGAAGGGAAGTAGAGTTGTCACT | SEQ ID NO: 4 |
| c-Fos Forward primer | CGAAGGGAACGGAATAAGATG | SEQ ID NO: 5 |
| c- Fos Reverse primer | GCTGCCAAAATAAACTCCAG | SEQ ID NO: 6 |
| DC-STAMP Forward primer | GGGAGTCCTGCACCATATGG | SEQ ID NO: 7 |
| DC-STAMP Reverse primer | AGGCCAGTGCTGACTAGGATGA | SEQ ID NO: 8 |
| OC-STAMP Forward primer | CAGAGTGACCACCTGAACAAACA | SEQ ID NO: 9 |
| OC-STAMP Reverse primer | TGCCTGAGGTCCCTGTGACT | SEQ ID NO: 10 |
| TRAF6 Forward primer | AAAGCGAGAGATTCTTTCCCTG | SEQ ID NO: 11 |
| TRAF6 Reverse primer | ACTGGGGACAATTCACTAGAGC | SEQ ID NO: 12 |

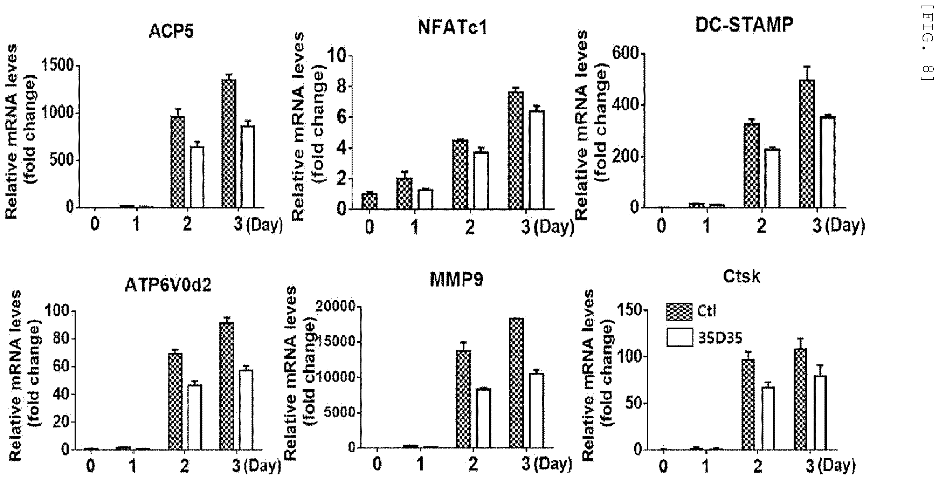

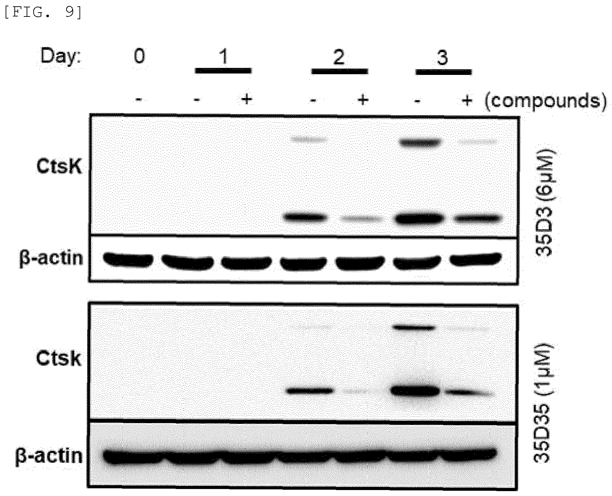

As a result of RT-PCR, an amount of mRNA expression of each of the osteoclast-specific genes, that is, AcP5 (TRAP), NFATcl, DC-STAMP, ATP6v0d2, MMP9 and CTSK in the group treated with 35D35 was decreased, as compared to the control not treated with 35D35 (Ctrl) (see FIG. 8, "M" = M-CSF treatment group). The graph of FIG. 8 is a result of normalization of transcription levels based on the expression level of the control on Day 0. In particular, it was confirmed that, when the 35D35 compound was used for treatment compared to the case where the 35D3 compound was used for treatment, Ctsk protein expression was strongly inhibited even at a low concentration (see FIG. 9). From the above results, it was confirmed that 35D35 compound could inhibit the differentiation of osteoclasts even in a smaller amount than the 35D3 compound, therefore, it is expected that 35D35 may function as an effective bone metabolism inhibitor.

### 2. Inhibitory effects of the compound represented by Formula 4 on osteoclast differentiation

### 1) Isolation of mouse bone marrow cells and differentiation of osteoclasts

The bone marrow of a 12-week-old female mouse was isolated from the hip bone, femur and tibia, followed by culturing the same in α-MEM medium (Gibco, 12561-056, 10% fetal bovine serum, 1% penicillin/streptomycin) containing macrophage colony stimulating factor (M-CSF, PeproTech, cat# 315-02, 30 ng/ml) for 3 days in order to isolate only monocytes. The isolated monocytes were treated with RANKL (PeproTech, cat# 315-11, 50 ng/ml) to induce osteoclast differentiation. Then, 35D3 (PPOA) was also used for treatment, followed by culturing the cells for 3 days or 5 days.

### 2) Confirmation of osteoclast differentiation inhibitory ability through TRAP staining and analysis

1 × 10⁴ monocytes isolated from the mouse bone marrow were put into a 96-well plate and treated with RANKL to confirm the effect of 35D3 (PPOA) (3 µM) on osteoclast differentiation ability.

The activity of tartrate-resistant acid phosphatase (TRAP), which has increased expression during osteoclast differentiation, was confirmed using a TRAP activity assay kit (Sigma-Aldrich, 387A-1kt), which is shown in FIG. 16.

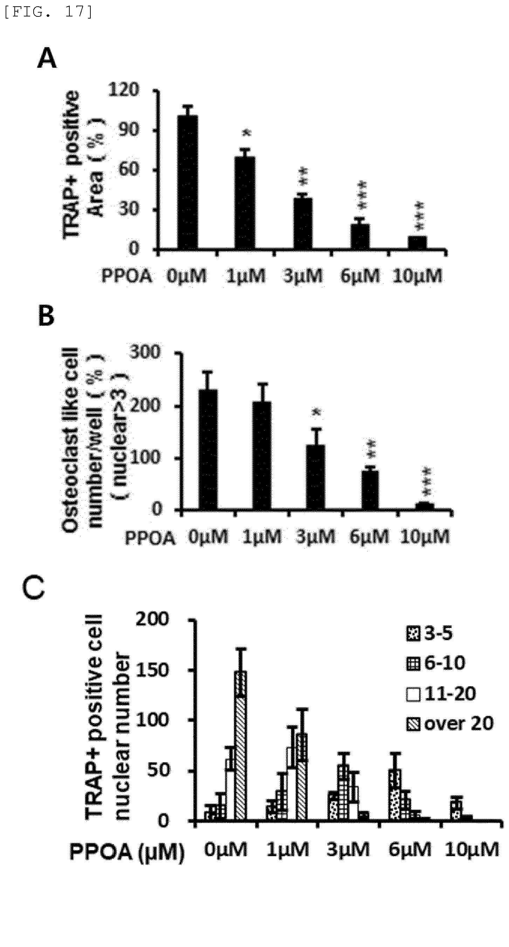

After staining, the multinuclear cells formed during osteoclast differentiation were observed under a microscope, and 12 images per well were photographed and combined into one well image, and a differentiation area ratio per total area was confirmed using Image J software. The differentiation area ratio per total area is shown in FIG. 17A, and the area of TRAP-positive cells (nuclear number > 3) was compared with DMSO (100%) and presented as a relative value.

Further, the number of differentiated osteoclasts was counted and determined, and this was shown in FIG. 17B, and cells in which the number of cell nuclei contained in the differentiated osteoclasts is in the range of 3-5, 6-10, 11-20, and 21 or more, were classified and shown in FIG. 17C.

Referring to FIGS. 16 and 17, it was confirmed that, when the compound of the present invention was used for treatment, all the differentiation area, the number of osteoclasts and giant multinuclear cells were significantly reduced as compared to the group in which osteoclasts were differentiated normally. These results may demonstrate the osteoclast differentiation inhibitory ability of 35D3 (PPOA).

### 3) Confirmation of actin-ring formation inhibition and bone resorption inhibitory effect

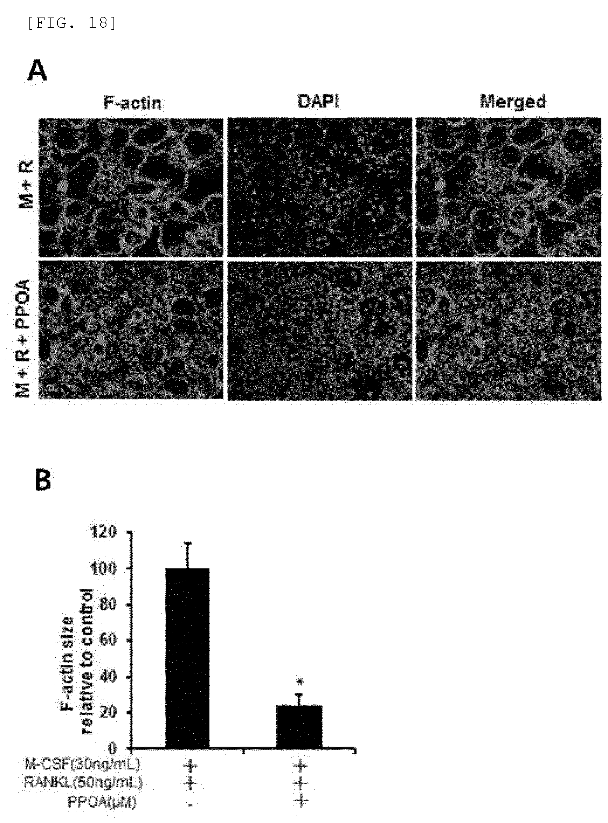

In order to determine the activity of differentiated osteoclasts, an ability of differentiating into multinuclear cells was measured through F-actin staining. 5 × 10⁴ mouse bone marrow cells per well was put into a 24-well plate in which glass coated with L-Lysine was placed (Corning, 354085). At the same time, the cells were treated with 6 uM of 35D3 (PPOA) and then cultured for 4 to 5 days. The differentiated cells were fixed with 4% formaldehyde for 10 minutes and washed with PBS. For an antigen-antibody reaction, the cells were subjected to a reaction in 0.1% Triton-PBS for 20 minutes, followed by a reaction with 1% BSA for 1 hour. The F-actin antibody (Thermo Fisher Scientific, A12379) was reacted for 1 hour and then washed. Cell nuclei were stained through DAPI (4',6-Diamidino-2-Phenylindole, Dilactate, Thermo Fisher Scientific, D3571) staining, and then, photographed under a microscope. As a result, it was confirmed that the size of F-actin was different depending on the presence or absence of 35D3 (PPOA) treatment (see FIG. 18). From these results, it was demonstrated that 35D3 (PPOA) could inhibit differentiation into multinuclear cells.

Further, the bone resorption ability was confirmed using a bone resorption assay kit (Cosmo Bio, CSR-BRA). Mouse bone marrow cells were seeded on a bone resorption assay plate 48 (2 × 10⁴ cells/well) containing 30 ng/Ml of M-CSF. After 24 hours, mouse bone marrow cells were treated with or without 6 µM 35D3 (PPOA) under stimulation of 30 ng/mL of M-CSF and 100 ng/mL of RANKL until mature osteoclasts were formed. The next day, the culture supernatant from the cells was collected on a 96-well black polypropylene micro-well plate (Thermo Fisher Scientific Nunc, Waltham, MD, USA). After mixing 50 µL of 0.1 N of NaOH, fluorescence intensity was measured with a fluorescence plate reader (Molecular Devices, San Jose, CA, USA-model: SpectraMax i3x); and the excitation and emission wavelengths were 85 nm and 535 nm, respectively. The absorption area was calculated in consideration of 10 randomly selected pictures per well taken at 10 × magnification using Image J software (https://imagej.nih.gov/ij).

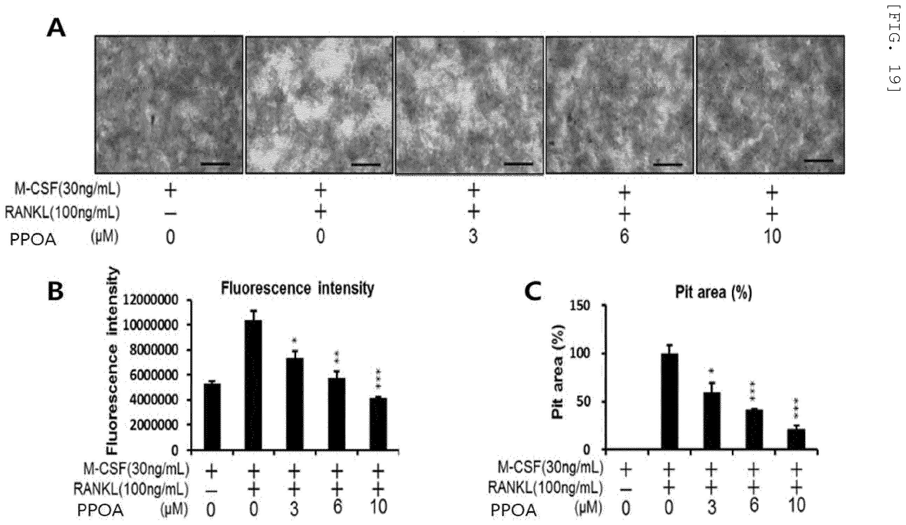

FIG. 19A shows visualization of the bone resorption area using an optical microscope. FIG. 19B shows the fluorescence intensity measured at an excitation wavelength of 485 nm and an emission wavelength of 535 nm, respectively. Further, FIG. 19C shows results of calculating the bone resorption pits area. The proportion of bone resorption area showed a significant decrease after exposure to 3, 6, and 10 µM of 35D3 (PPOA).

### Confirmation of osteoblast differentiation effects

After collecting cells from C57BL/6J mouse calvaria at the age of 3 days, the cells were seeded on a 96-well plate in an amount of 4 × 10³cells per well, and then treated with 100 ng/ml of hBMP2 as a differentiation inducer. Simultaneously with the differentiation inducer treatment, the cells were treated with the 35D35 compound and cultured for 7 days, followed by ALP staining to assess differentiation influence. Specific experimental methods and results are as follows.

### 1) Isolation of mouse skull cells and differentiation of osteoblasts

Precursor cells isolated from the mouse skull were treated with hBMP2 (Bone morphogenic protein 2, Sino biological, 10426-HNAE, 100 ng/ml) to induce differentiation into osteoblasts. Differentiated osteoblasts could be discriminated through alkaline phosphatase (ALP) staining as an osteoblast-specific protein. When the progenitor cells were treated with hBMP2, each of the 35D35 compounds was also used for treatment, followed by culturing the cells for 7 days while changing the medium every 2 to 3 days.

### 2) ALP (alkaine phosphatase) staining for analysis of osteoblast differentiation ability

4 × 10³ osteoblast progenitor cells were put into a 96-well plate and treated with hBMP2 to induce differentiation of osteoblasts. At this time, the cells were treated with 35D35 and then cultured for 7 days.

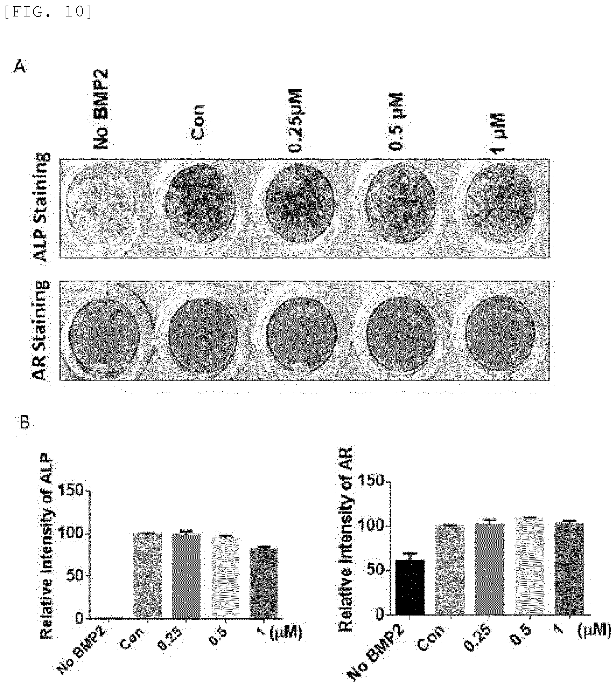

For the analysis of osteoblast differentiation ability, the medium was discarded 7 days after induction of differentiation to confirm the activity of ALP, which shows increased expression in the early stage of osteoblast differentiation, the remaining product was washed once with a Hanks' Balanced Salt Solution (HBSS, welgene) and put into 70 % cold ethanol, followed by fixing the cells for 1 hour. After discarding the ethanol, the remaining product was washed with HBSS, followed by adding a NBT solution (Sigma, B1911) in an amount of 100 µl per well and staining for 15 minutes. By washing twice with water, the remaining dye was eliminated. After ALP staining, a degree of color development was measured using Image J software, and then graphed for comparison. As a result, it was confirmed that 35D35 did not affect osteoblasts (see FIG. 10). FIG. 10 shows results of confirming the osteoblast differentiation ability by the 35D35 compound through ALP staining. Specifically, FIG. 10A shows results of confirming the differentiated osteoblasts through ALP staining, while FIG. 10B shows ALP intensity graph ("Con" = BMP2 alone treatment group),

### Confirmation of in vivo effects

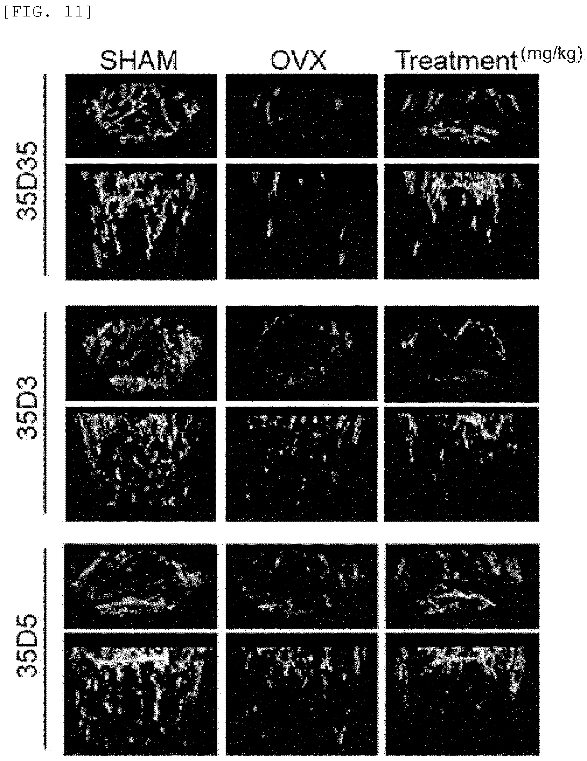

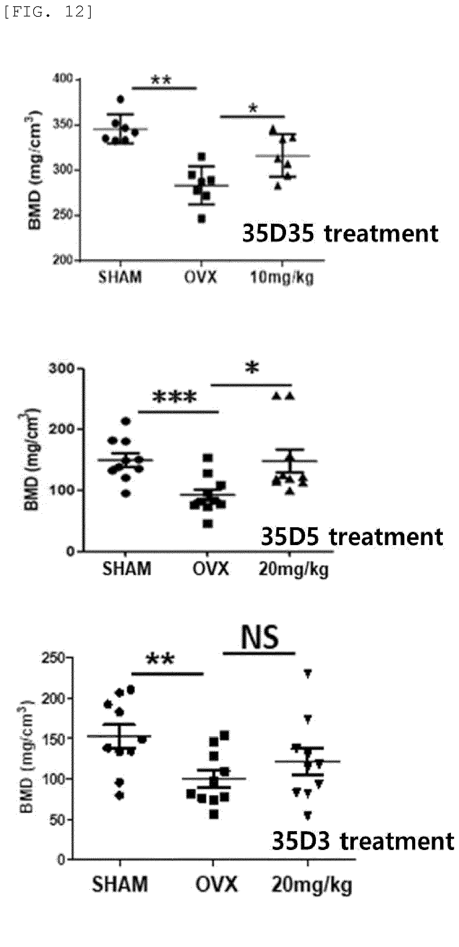

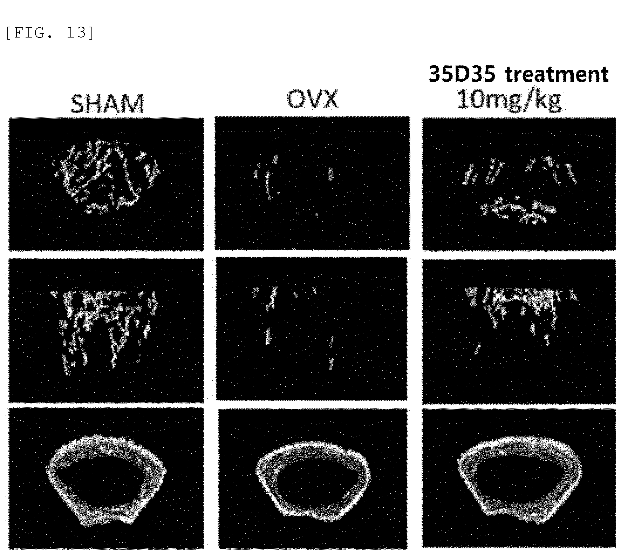

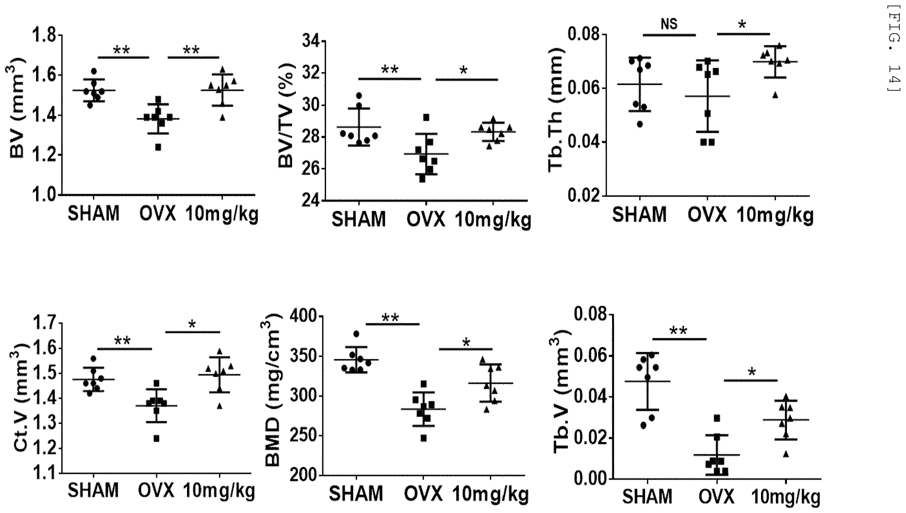

An ovariectomized mouse model (OVX, ovariectomized mouse) with osteoporosis due to estrogen deficiency after ovariectomy in 8-week-old female mouse were prepared. Compounds (35D35, 35D5, and 35D3), respectively, were injected intraperitoneally once every two days to the ovariectomized mouse model. After 4 weeks, the tibia of each mouse was analyzed for bone density by microcomputer tomography. As a result, it was observed that the OVX model injected with the 35D3 compound did not exhibit higher bone density and bone volume than the control (OVX), whereas the OVX model injected with the 35D35 and 35D5 compounds had higher bone density and bone volume than the control (OVX) (see FIGS. 11 to 14).

FIGS. 11 to 14 show the micro-computed tomography results (Sham: the control in which the ovaries were not removed) and graphs for analysis of the micro-computed tomography results with regard to the tibia of the mouse models (*P<0.05, **P<0.01, and ***P<0.001 vs. the control, OVX. BMD: trabecular bone mineral density).

From the above-described experimental results, it was confirmed that the compounds (35D35, 35D5, and 35D3) of the present invention could exhibit excellent osteoclast differentiation inhibitory effects, while not affecting osteoblast differentiation. Further, it could be presumed that the compound may inhibit the expression of osteoclast-related genes by inhibiting the expression of osteoclast-related genes including NFATc1 through molecular biological experiments. In addition, when each of the compounds (35D35, and 35D5) was added to treat the ovariectomized mouse model with osteoporosis, excellent inhibition of bone resorption ability was demonstrated. As such, the compound of the present invention may function as an effective bone metabolism inhibitor since excellent osteoclast differentiation inhibitory effects were confirmed.

## Claims

1. A pharmaceutical composition for preventing or treating bone diseases caused by hyperdifferentiation of osteoclasts, the pharmaceutical composition comprising a compound represented by Formula 1 below or a pharmaceutically acceptable salt thereof: 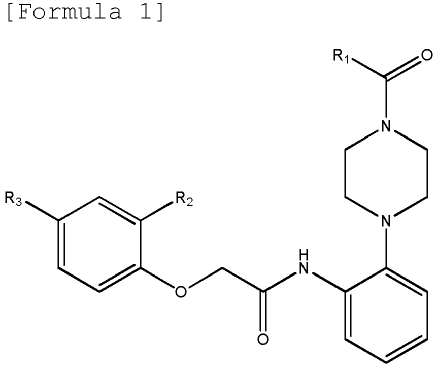 In Formula 1 above,
R₁ is C1 to C3 alkyl or phenyl; and
R₂ and R₃ are each independently H or halo.

2. The pharmaceutical composition according to claim 1, wherein R₁ is methyl, ethyl or phenyl.

3. The pharmaceutical composition according to claim 1, wherein R₁ is C1 to C3 alkyl, R₂ is halo and R₃ is H; or R₁ is phenyl, R₂ is H and R₃ is halo.

4. The pharmaceutical composition according to claim 1, wherein the compound represented by Formula 1 above is any compound represented by Formulae 2 to 4 below:

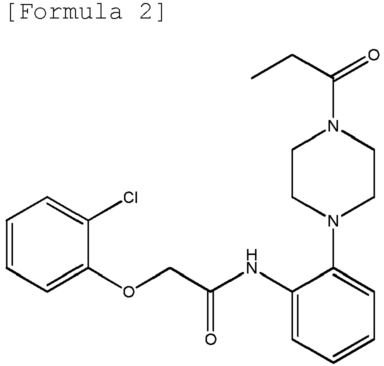

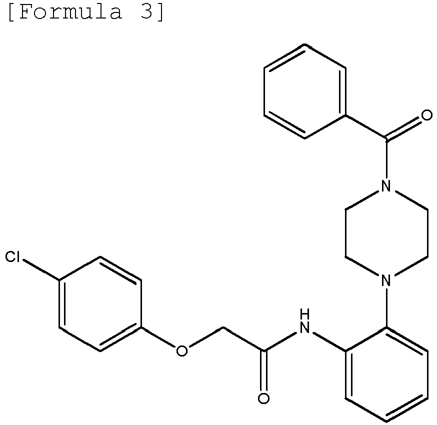

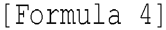

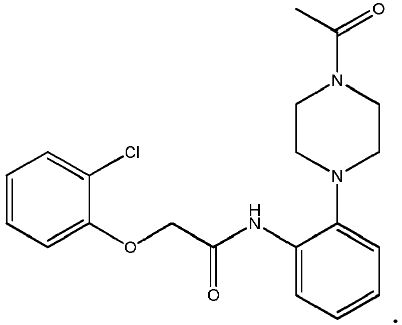

5. The pharmaceutical composition according to claim 1, wherein the bone disease is at least one selected from the group consisting of fracture, osteoporosis, rheumatoid arthritis, periodontitis, Paget's disease, osteomalacia, osteopenia, bone atrophy, osteoarthritis, and avascular femoral necrosis.

6. A health functional food for preventing or treating bone diseases caused by hyperdifferentiation of osteoclasts, the health functional food comprising a compound represented by Formula 1 below or a food acceptable salt thereof: 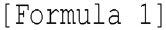 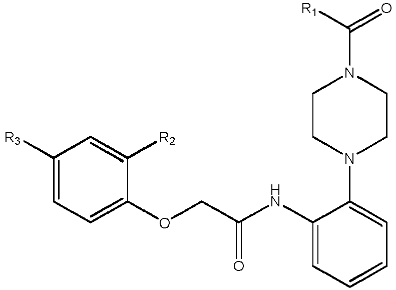 In Formula 1 above,
R₁ is C1 to C3 alkyl or phenyl; and
R₂ and R₃ are each independently H or halo.

7. The health functional food according to claim 6, wherein R₁ is methyl, ethyl or phenyl.

8. The health functional food according to claim 6, wherein R₁ is C1 to C3 alkyl, R₂ is halo and R₃ is H; or R₁ is phenyl, R₂ is H and R₃ is halo.

9. The health functional food according to claim 6, wherein the compound represented by Formula 1 above is any compound represented by Formulae 2 to 4 below:

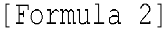

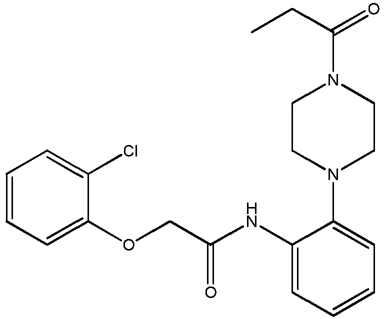

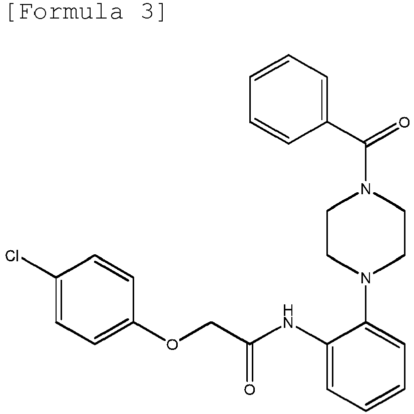

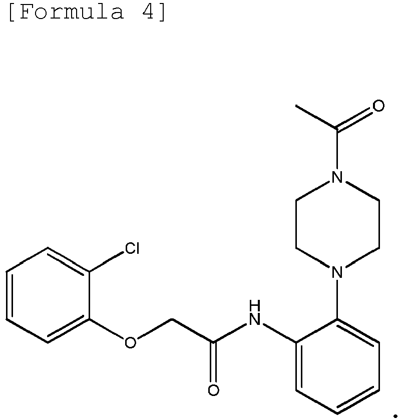

10. The health functional food according to claim 6, wherein the bone disease is at least one selected from the group consisting of fracture, osteoporosis, rheumatoid arthritis, periodontitis, Paget's disease, osteomalacia, osteopenia, bone atrophy, osteoarthritis, and avascular femoral necrosis.
